# EUROPEAN PATENT SPECIFICATION

(11) **EP 2 536 354 B1**
(45) Date of publication and mention of the grant of the patent: **08.08.2018**
(21) Application number: 11704191.3
(22) Date of filing: 16.02.2011
(51) Int. Cl.: A61F 2/24, A61F 2/90

(54) **EXPANDABLE MEDICAL IMPLANT**
EXPANDIERBARES MEDIZINISCHES IMPLANTAT
IMPLANT MÉDICAL EXTENSIBLE

(30) Priority: 22.02.2010 US 306564 P; 17.02.2010 DE 102010008362
(43) Date of publication of application: 26.12.2012
(73) Proprietor: Venus MedTech (HangZhou), Inc., Hangzhou, Zhejiang (CN)
(72) Inventor: GOETZ, Wolfgang, 93051 Regensburg (DE); LIM, Hou-Sen, 455234 Singapore (SG)
(74) Representative: Zenz Patentanwälte Partnerschaft mbB
(86) International application number: PCT/EP2011/000735
(87) International publication number: WO 2011/101126

(56) References cited:
- EP-A1- 1 767 240
- WO-A2-2008/024712
- DE-A1-102008 013 948
- US-A1- 2004 034 402
- US-A1- 2005 149 168
- US-A1- 2007 100 435
- US-A1- 2009 222 082
- US-A1- 2010 016 943
- US-B1- 7 258 697

## Description

The present invention relates to a medical implant having the features of claim 1. It further relates to a set having the features of claim 7. Medical implants such as stents, implantable cardiac valve supporting devices, and the like, are usually folded or collapsed, respectively, before they are inserted into the patient's body and are then inserted, e.g., shifted, to the implantation site by means of suitable tools. The unfolding of the folded or collapsed implant is performed at the implantation site, either actively or passively.

Thereby, it can be necessary to re-fold or re-collapse, respectively, an implant which has been placed incorrectly in the target organ (in a vessel or another organ, see above) or to reduce the diameter of the implant in order to re-implant it at or in the originally intended site.

From US 2005/0149168 A1 a stent to be deployed on a bend is known.

From EP 1 767 240 A1 a stent comprising ring-shaped units and a connection portion is known.

From US 7,258,697 B1 a stent with anchors to prevent vulnerable plaque rupture during deployment is known.

From US 2004/0034402 A1 a helical stent having a flexible transition zone is known.

From DE 10 2008 013 948 A1 a stent which is reduceable again in its diameter from a expanded state in a controlled manner is known.

An object of the present invention is to propose a suitable design or embodiment of the expandable medical implant.

This object according to the present invention is solved by the feature combination according to claim 1.

Thus, according to the invention, there is proposed an expandable and/or collapsible implant, in particular a stent, comprising at least one supporting means which is suited for supporting the implant at or on an implantation site. The implant is expandable from a respective first diameter to a respective second diameter and/or are collapsible from the second diameter to the first diameter. The supporting means comprises bars which are connected to each other by means of connecting sections; and at least one post for connecting the supporting means with at least one other structure of the implant.

Advantageous embodiments and developments of the present invention are subject-matter of the dependent claims.

The at least one geometrical characteristic in which at least two of the connecting sections differ from another is, in some of the embodiments according to the invention, understood as a width of the respective connecting sections.

In some of the embodiments according to the invention, the geometrical characteristic refers to a width of at least one section of the connecting sections in a longitudinal direction of the implant and/or in a direction present at a right angle thereto (lateral direction). In an implant which is perfused by bodily fluids after its implantation, the longitudinal direction of the implant can correspond to the direction of the (main) perfusion. The lateral direction of the implant can be a direction in which a (main) expansion of the supporting means takes place (e.g., at the implantation site in the patient's body or before the implantation in a laboratory without any external application of force to the supporting means).

The implant according to the invention has a first width d1 of a first connecting section, which is the closest connecting section to a post. The term "which is the closest connecting section to a post" is to be understood as "having the smallest distance to a post". Thereby, this width is the smallest width of all widths of the connecting sections.

In some of the embodiments of the implant according to the invention, a second connecting section has a second width d2. Width d2 is larger than width d1.

In some embodiments, the second connecting section has the next smaller distance to the post. It is thus adjacent to the first connecting section and has a larger distance to the considered post than the first connecting section.

In some of the embodiments of the implant according to the invention, a third connecting section has a third width d3. Width d3 is larger than width d2. It is thus adjacent to the second connecting section; however, it is not adjacent to the first connecting section and has a larger distance to the considered post than the first connecting section and the second connecting section.

In some of the embodiments of the implant according to the invention, a post is understood as a section of the supporting means which substantially is not curved, i.e., extends linearly.

In some embodiments, a connecting section is understood as a section of the connecting means which connects two or more bars with each other und which is more curved than at least one of the bars.

In some embodiments, a connecting section is understood as a section of the supporting means being a point of curvature in the sense of a curve progression. Thereby, the point of curvature of the connecting section is the point which is ever located most next to an end of the implant - with respect to the longitudinal direction thereof.

In some of the embodiments of the implant according to the invention, the implant is a cardiac valve - in particular an artificial one or one which has been manufactured from animal tissue.

In certain embodiments according to the invention, bars comprised by the supporting means which connect a rod or post with the adjacent connecting sections are shorter than other bars. The shorter bars contribute to forming a slit that reaches from an end of the supporting means to the adjacent end of the post. The slit has a length of L2 (from its open end to its closed end).

From the opposite end of the slit to the adjacent end of string outlet or aperture 10 a distance having a length L3 is provided. Between the closed end of the slit and the centre of string outlet or aperture a distance having a length L4 is provided.

The open end of the slit may be spaced from the centre of the outlet or aperture by the sum of L2 and L4.

In a preferred embodiment, L1 is between 2.5 and 3.5 times as long as L2, preferably 3 times as long. L1 is the length of the supporting means in a distal-proximal direction thereof.

In some embodiments, L2 is 2 times (or between 1.5 and 2.5 times) the length of L4.

In certain embodiments, L2 is 3 times (or between 2.5 and 3.5 times) the length of L4.

A - preferably controlled - unfolding and/or re-folding of the implant (which can also include expanding and returning the implant to a reduced diameter) can be effected by means of a known catheter. The catheter can be designed as is described in US 2007/0100427 A1 by Perouse or in US 2005/0075731 A1 by Artof et al..

Regarding its basic design, the implant can be constructed as is, for example, described in DE 10 2008 013 948 A1 by the applicant of the present invention.

The implant can be self-expanding, for example, it can be formed from or with a memory material, in particular nitinol, or materials which comprise nitinol. The implant can also be partly self-expanding, partly by the use of an expanding means. The implant can exclusively be non-self-expanding. The implant can be foldable or collapsible, respectively; the implant can be non-foldable or non-collapsible, respectively.

The implant can comprise a biocompatible material, in particular a biocompatible stainless steel. The material can be bio-absorbable.

The implant can be designed with or without a means for encompassing or sandwiching parts of native valve sections (in particular heart valve leaflets). In particular, the implant can be designed with or without sections rising up or lowering down due to temperature and memory effect.

According to the invention, a set is proposed which comprises at least one implant according to the invention, in particular a stent; and at least one catheter.

The advantages achievable herewith correspond to at least those of the implant according to the invention.

The following advantages may be found in certain embodiments according to the invention.

By using the implant according to the invention, an expansion thereof is, according to the invention, possible without distorting or warping, respectively, or undulating or waving, respectively, the supporting structure. In some embodiments according to the invention, this can primarily enable a multiple, however, at least a twofold, re-expansion following a happened reduction of the cross-section. The desired expansion behavior of the supporting means is maintained in such a case, too. A distortion or warping, respectively, or an undulation or waving, respectively, does not take place in such a case, too. The latter advantageously allows for a precise positioning of the implant. It can further ensure the more accurate residing of the supporting means and, thus, of the implant against the tissue of the implantation site.

Further, the likelihood of any undue or adverse stress concentration within the solid parts of the implant can advantageously be avoided or minimized.

The invention is exemplarily illustrated by means of the appended figures. In the figures, same or similar structures are denoted by the same reference numerals throughout the figures, wherein:
- Fig. 1: shows a medical implant according to the present invention in an expanded state which is expandable and can be reduced in its diameter by use of a means;
- Fig. 2: shows the medical implant of Fig. 1 in a non- or less expanded state;
- Fig. 3: shows enlarged sections of the medical implant of Fig. 1;
- Fig. 4: shows enlarged sections of a supporting means of the implant of Fig. 1 to 3 according to the invention; and
- Fig. 5: shows an enlarged section of a supporting means of the implant according to the invention.

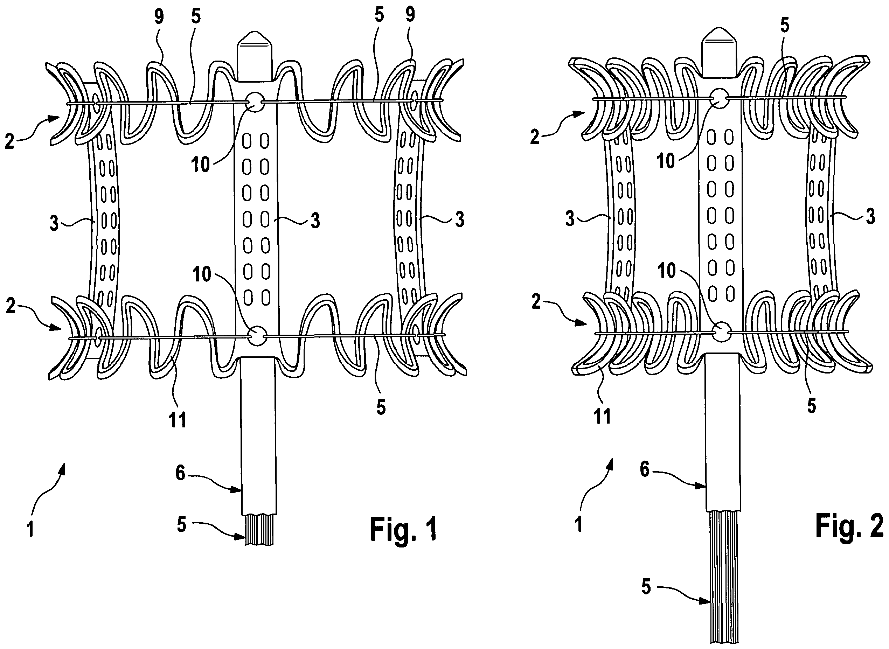

**Fig. 1** shows an implant 1 which is expandable and can be reduced in its diameter. The diameter thereby refers to a plane perpendicular to a longitudinal axis of the medical implant 1. The longitudinal direction also corresponds to the direction of the extension of the catheter 6 shown in Fig. 1. The implant 1 comprises two circular supporting means or rings 2. The supporting means 2 are connected to rods or posts 3. In some embodiments, the supporting means 2 can - additionally or alternatively or exclusively - fulfill the function of a guiding means for reins 5. The reins 5 form part of a catheter 6 and serve for applying force or tension or stress, respectively, to the supporting means 2 for the purpose of expanding or folding the implant in a targeted manner. In the example of Fig. 1, the supporting means 2 are each designed in form of an outwardly half-open channel, through which the reins 5 are guided. The half-open channel is opened in a direction away from the center of the implant 1. However, the channel can also be designed in a form open to the implant or to another direction.

In the example of Fig. 1, the supporting means 2 are interrupted by posts 3, i.e. the posts 3 are integrated into the supporting means 2 such that they form sections of the supporting means 2.

In the embodiment of the implant 1 according to the invention of Fig. 1, the supporting means 2 have (round or differently shaped, e.g., oval, rectangular, elliptic, and so on) passage means or apertures 10. In the embodiment of the implant 1 according to the invention, these serve as a passage for the reins 5.

Furthermore, the implant 1 can also comprise a number of guiding means other than two, for example, one, three, four or more guiding means. The supporting means 2 can be arranged circularly, however, they can also be arranged non-circularly. The supporting means 2 can be formed integrally with the implant; however, they can also be fabricated separately. The supporting means 2 can have the form of a wave or undulation, respectively; however, they can also be fabricated in any other form, in particular, a non-wavy or non-undulating form.

Independent of all other features, the implant 1 can be fabricated from flat material, e.g., a material which has been cut with a laser, wherein, e.g., after having designed a pattern in the flat material, the material is reformed into a tube (optionally by connecting, such as welding, longitudinal sides of the former flat material lane or web, respectively). However, the implant 1 can also be fabricated from a tubular material directly.

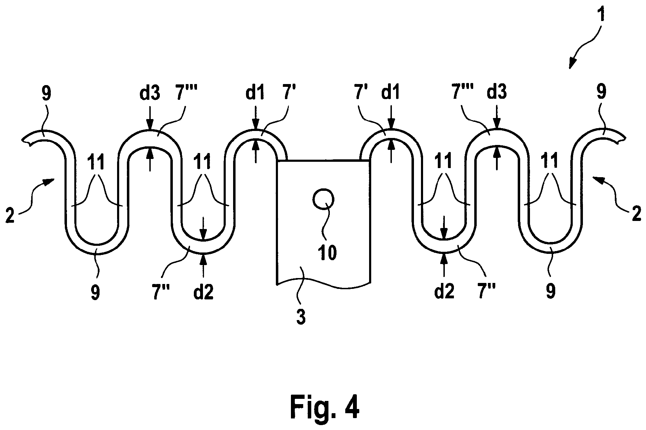

The supporting means 2 of the implant 1 consist of a plurality of bars 11 which are each connected to another by means of connecting sections 9. According to the invention, the connecting sections 9 differ in their design. However, as the latter is not shown in Fig. 1, reference is made to Fig. 4.

**Fig. 2** shows the implant 1 of Fig. 1. Two reins 5 have been led around the implant 1 and return back to the catheter 6 through the respectively same passage means or apertures 10. The reins 5 apply a tension or stress, respectively, on the implant 1 and the implant 1 is not completely expanded or unfolded, respectively. The diameter of the implant 1 has been reduced.

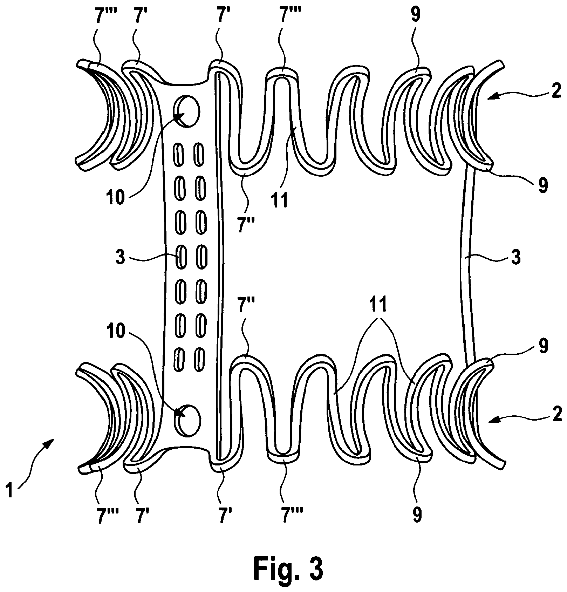

**Fig. 3** shows an enlarged section of the medical implant of Fig. 1. In this enlargement, connecting sections 7', 7", 7''' and 9 can be seen. All of them connect bars 11 which are arranged there between.

**Fig. 4** shows a detail of a supporting means 2 of the implant 1 according to the invention. For illustration purpose, this detail of the supporting means 2 is shown as an even structure in Fig. 4.

Connecting means 7' are shown which are each followed by a bar 11 and subsequent connecting sections 7". Those are again followed by another bar 11 which is in turn followed by another connecting section 7"'. Respective bars 11 and, finally, connecting sections 9 follow the connecting sections 7"' at both ends of the post 3.

Fig. 4 shows that the connecting sections 7', 7" and 7'" each have widths or thicknesses d1, d2 and d3. Thereby, in the presentation of Fig. 4, width d1 is smaller than width d2 and width d2 is smaller than width d3. That means: d1 < d2 < d3.

In the embodiment according to the invention shown in Fig. 4, the connecting section is an apex of curvature or comprises such an apex of curvature.

The difference of the widths d1, d2 and d3 is present in a direction which extends in parallel to a longitudinal axis of the medical implant 1.

In other embodiments according to the invention of the supporting means according to the invention, the differences are present in another direction, e.g., in a direction which does not extend in parallel to a longitudinal axis of the medical implant during a state of use (e.g., before an extracorporeal expansion) or in turn in another direction. This other direction can be a direction perpendicular to a longitudinal axis of the medical implant 1. Moreover, this other direction can be any other direction.

The widths of the connecting sections 9 can, e.g., correspond to width d3. However, the connecting sections 9 can also have any other width. In particular, the connecting sections 9 can have a uniform width.

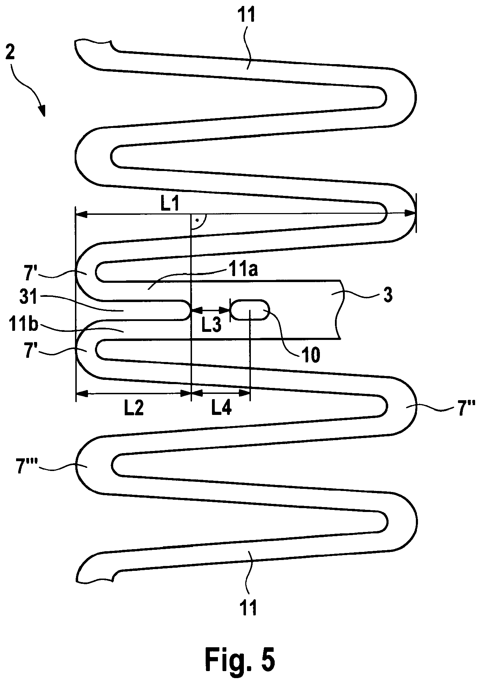

**Fig. 5** shows an enlarged section of the supporting means 2 of the implant 1 according to one embodiment of the invention. In the example of Fig. 5, the supporting means has a length of L1 (from the distal end to the proximal end).

As can be seen from Fig. 5, the supporting means 2 comprises bars 11a and 11b which connect a rod or post 3 comprising an (oval) string outlet or aperture 10 with corresponding adjacent connecting sections 7', respectively. Bars 11a and 11b which can be considered as to merge with the post 3 (in contrast to other bars 11) are shorter than other bars 11. In fact, bars 11a and 11b contribute to forming a slit 31 that reaches from an end of the supporting means 2 shown at the left-hand side of the representation of Fig. 5 to the left-hand end of rod or post 3. In Fig. 5, the slit has a length of L2.

As can further be derived from Fig. 5, between the right-hand end of slit 31 and the left-hand end of string outlet or aperture 10 a distance having a length L3 is provided. The distance L3 may be filled with a solid part of post 3.

Between the right-hand end of slit 31 and the centre of string outlet or aperture 10 a distance having a length L4 is provided.

The left-hand end of the supporting means 2 of Fig. 5 may be spaced from the centre of the outlet or aperture 10 by the sum of L2 and L4.

As regards to the relation of L1, L2, L3, and L4, it is noted that in one preferred embodiment of the invention, L1 is between 2.5 and 3.5 times as long as L2, preferably 3 times as long.

In some embodiments, L2 is 2 times (or between 1.5 and 2.5 times) the length of L4.

In certain embodiments, L2 is 3 times (or between 2.5 and 3.5 times) the length of L3.

As regards Fig. 5, it is noted that the left-hand end of the supporting means 2 may be the distal or proximal end of the supporting means 2 and/or of the implant 1.

It is to be noted that the features described with reference to Fig. 5 may be embodied in an implant according to the invention without necessarily comprising also features described with regards to Fig. 1 to 4.

## Claims

1. An implant (1) comprising at least one circular supporting means (2) which is suited for supporting the implant (1) at or on an implantation site, wherein the implant (1) is expandable from a first diameter to a second diameter and/or is collapsible from the second diameter to the first diameter, wherein the supporting means (2) is designed in the form of an undulation comprises:
- bars (11, 11a, 11b) which are connected to each other by means of connecting sections (7', 7", 7"', 9);
- at least one post (3) for connecting the supporting means (2) with at least one other structure of the implant (1), wherein at least two of the connecting sections (7', 7", 7"', 9) of one supporting means (2) differ in at least one geometrical characteristic,
and wherein the geometrical characteristic of the supporting means (2) is a width of at least one section of the connecting sections (7', 7", 7"', 9), wherein a first connecting section (7'), which is the closest connecting section connected to one post (3) by a single bar, has a first width d1, wherein the width d1 is the smallest width of all widths of the connecting sections (7', 7", 7"', 9),
**characterized in that**
a second connecting section (7") having the next smaller distance to the post (3) has a second width d2, wherein width d2 is larger than width d1.

2. An implant (1) according to claim 1, wherein a third connecting section (7"') having in turn the next smaller distance to the post (3) has a third width d3, wherein width d3 is larger than width d2.

3. An implant (1) according to anyone of the preceding claims being a cardiac valve.

4. An implant (1) according to anyone of the preceding claims, comprising a shape memory material, in particular nitinol, or consisting thereof.

5. An implant (1) according to anyone of the preceding claims, wherein the supporting means (2) is designed in form of an outwardly half-open channel.

6. An implant (1) according to anyone of the preceding claims, wherein bars (11a, 11b) comprised by the supporting means (2) which connect a post (3) with the adjacent connecting sections (7') are shorter than other bars (11) and form a slit from an end of the supporting means (2) to the adjacent end of the post (3).

7. A set comprising at least one expandable and/or collapsible implant (1), in particular a stent, according to anyone of claims 1 to 6 and at least one catheter (6) or other delivery device for inserting the medical implant (1).

## Patentansprüche

1. Ein Implantat (1) aufweisend zumindest ein kreisförmiges Unterstützungsmittel (2), welches zum Unterstützen des Implantats (1) bei oder auf einer Implantationsstelle geeignet ist, wobei das Implantat (1) ausgehend von einem ersten Durchmesser hin zu einem zweiten Durchmesser erweiterbar und/oder ausgehend von dem zweiten Durchmesser zu dem ersten Durchmesser kollabierbar ist, wobei das Unterstützungsmittel (2) in einer Wellenform ausgebildet ist und aufweist:
- Stangen (11, 11a, 11b), welche miteinander mittels Verbindungsabschnitten (7', 7", 7"', 9) verbunden sind,
- zumindest einen Pfosten (3) zum Verbinden des Unterstützungsmittels (2) mit zumindest einer anderen Struktur des Implantats (1), wobei zumindest zwei der Verbindungsabschnitte (7', 7", 7"', 9) des Unterstützungsmittels (2) sich in zumindest einer geometrischen Eigenschaft unterscheiden,
und wobei die geometrische Eigenschaft des Unterstützungsmittels (2) eine Breite zumindest eines Abschnittes der Verbindungsabschnitte (7', 7", 7"', 9) ist,
wobei ein erster Verbindungsabschnitt (7'), welcher der engste mit einem Pfosten (3) durch eine einzelne Stange verbundene Verbindungsabschnitt ist, eine erste Breite d1 aufweist, wobei die Breite d1 die kleinste Breite sämtlicher Breiten der Verbindungsabschnitte (7', 7", 7"', 9) ist,
**dadurch gekennzeichnet, dass** ein zweiter Verbindungsabschnitt (7") mit dem nächstkleineren Abstand zu dem Pfosten (3) eine zweite Breite d2 aufweist, wobei die Breite d2 größer als die Breite d1 ist.

2. Ein Implantat (1) nach Anspruch 1, wobei ein dritter Verbindungsabschnitt (7''') mit dem wiederum nächstkleineren Abstand zu dem Pfosten (3) eine dritte Breite d3 aufweist, wobei die Breite d3 größer als die Breite d2 ist.

3. Ein Implantat (1) nach einem der vorhergehenden Ansprüche, wobei das Implantat eine Herzklappe ist.

4. Ein Implantat (1) nach einem der vorhergehenden Ansprüche, aufweisend oder bestehend aus einem Material mit einem Formgedächtnis, insbesondere Nitinol.

5. Ein Implantat (1) nach einem der vorhergehenden Ansprüche, wobei das Unterstützungsmittel (2) in Form eines nach außen halboffenen Kanals ausgebildet ist.

6. Ein Implantat (1) nach einem der vorhergehenden Ansprüche, wobei die Stangen (11a, 11b) des Unterstützungsmittels (2), welche einen Pfosten (3) mit dem benachbarten Unterstützungsabschnitt (7') verbinden, kürzer als andere Stangen (11) sind und von einem Ende des Unterstützungsmittels (2) zu dem benachbarten Ende des Pfostens (3) einen Schlitz ausbilden.

7. Ein Set aufweisend zumindest ein erweiterbares und/oder kollabierbares Implantat (1), insbesondere einen Stent, nach einem der Ansprüche 1 bis 6, und zumindest einen Katheter (6) oder eine andere Zuführeinrichtung zum Einführen des medizinischen Implantats (1).

## Revendications

1. Implant (1) comprenant au moins un moyen support circulaire (2) qui est apte à supporter l'implant (1) au niveau d'un ou sur un site d'implantation, l'implant (1) étant extensible depuis un premier diamètre jusqu'à un second diamètre et/ou étant rétractable du second diamètre au second diamètre, le moyen support (2) étant conçu sous la forme d'une ondulation et comprenant :
- des barres (11,11a,11b) qui sont connectées les unes aux autres au moyen de deux sections de connexion (7',7", 7'", 9) ;
- au moins une broche (3) pour connecter le moyen support (2) à au moins une autre structure de l'implant (1), au moins deux des sections de connexion (7',7'', 7"', 9) d'un moyen support (2) différant par au moins une caractéristique géométrique ;
et la caractéristique géométrique du moyen support (2) étant une largeur d'au moins une section parmi les sections de connexion (7',7", 7"', 9),
une première section de connexion (7') qui est la section de connexion la plus proche connectée à une broche (3) par une seule barre ayant une première largeur d1, la largeur d1 étant la plus courte largeur de toutes les largeurs des sections de connexion (7',7",7'",9),
**caractérisé en ce qu'**une deuxième section de connexion (7") présentant la distance suivante la plus proche du poteau (3) a une seconde largeur d2, la largeur d2 étant supérieure à la largeur d1.

2. Implant (1) selon la revendication 1, dans lequel une troisième section de connexion (7"') présentant à son tour la plus courte distance suivante par rapport à la broche (3) a une troisième largeur d3, la largeur d3 étant supérieurs à la largeur d2.

3. Implant (1) selon l'une quelconque des revendications précédentes étant une valve cardiaque.

4. Implant (1) selon l'une quelconque des revendications précédentes, comprenant un matériau à mémoire de forme, en particulier du nitinol, ou composé de ce dernier.

5. Implant (1) selon l'une quelconque des revendications précédentes, dans lequel le moyen support (2) est conçu sous la forme d'un canal à moitié ouvert vers l'extérieur.

6. Implant (1) selon l'une quelconque des revendications précédentes, dans lequel des barres (11a, 11b) faisant partie du moyen support (2) qui connecte une broche (3) aux sections de connexion adjacentes (7') sont plus courtes que d'autres barres (11) et forment une fente depuis une extrémité du moyen support (2) jusqu'à l'extrémité adjacente de la broche (3).

7. Ensemble comprenant au moins un implant extensible et/ou rétractable (1), en particulier un stent, selon l'une quelconque des revendications 1 à 6 et au moins un cathéter (6) ou un autre dispositif de délivrance pour insérer l'implant médical (1).
